# EUROPEAN PATENT SPECIFICATION

(11) **EP 3 207 906 B1**
(45) Date of publication and mention of the grant of the patent: **29.01.2025**
(21) Application number: 16206908.2
(22) Date of filing: 31.01.2014
(51) Int. Cl.: A61F 13/534, A61F 13/535, A61F 13/537, A61F 13/53, A61F 13/539, A61F 13/531

(54) **MULTI-LAYER ABSORBENT MATERIAL**
MEHRSCHICHTIGES ABSORBIERENDES MATERIAL
MATÉRIAU ABSORBANT MULTICOUCHE

(43) Date of publication of application: 23.08.2017
(62) Divisional of application: 14153470.1
(73) Proprietor: Ontex BV, 9255 Buggenhout (BE)
(72) Inventor: WEBER, Ainas, 56727 Mayen (DE); DE POORTER, Annick, 9320 Erembodegem - Aalst (BE)
(74) Representative: Larangé, Françoise

(56) References cited:
- EP-A1- 0 947 549
- EP-A1- 0 976 373
- EP-A1- 1 679 054
- EP-A1- 1 870 067
- EP-A1- 2 163 266
- EP-A1- 3 025 687
- EP-A2- 0 217 666
- WO-A1-2005/007962
- WO-A1-2013/152809
- WO-A1-2014/068487
- WO-A1-2014/145326
- WO-A1-98/55295
- WO-A2-99/17679
- JP-A- 2008 161 584
- JP-A- 2013 072 151
- JP-A- H06 136 654
- US-A- 5 865 824
- US-A1- 2001 014 797
- US-A1- 2007 142 803
- US-A1- 2010 280 479
- US-A1- 2011 270 204
- US-A1- 2012 203 191
- US-A1- 2014 005 622

## Description

### TECHNICAL FIELD

The present invention relates to a multi-layer absorbent material. More particularly, the invention relates to a layered, composite absorbent material with individual layers which are constructed and arranged to selectively cooperate to provide desired performance parameters in the composite, layered structure, to methods for manufacturing said multi-layer absorbent material, as well as to the use of a multi-layer absorbent material according to the invention in an absorbent article, particularly a diaper. Such a multi-layer material provides improved ability to absorb and retain fluids and consequently, prevents excessive rewetting and leakage. The present invention is of particular importance to the field of disposable hygiene products, in particular diapers.

### BACKGROUND

Performance objectives of disposable absorbent articles, such as infant diapers, include no product leakage, dry feel to the wearer, and a comfortable fit throughout the product life. Accordingly, absorbent articles typically contain an absorbent core to provide liquid handling and other absorbent functionalities required to meet the product performance objectives. Absorbent cores of absorbent articles are commonly composed of wood pulp fibers, and superabsorbent polymer particles are often distributed in the absorbent cores to enhance the liquid absorbent capacity. Absorbent articles frequently leak before the liquid absorbent capacity of the entire absorbent core is fully utilized. One problem resulting in leakage is the inability of the absorbent core to fully uptake liquids rapidly and completely when large amounts of liquids are discharged into the absorbent article. Another associated problem contributing to leakage is the inability of the absorbent core to move or distribute sufficient amounts of liquid between discharges from a target area portion of the absorbent article to more distal and more remote end regions of the absorbent core which have not been utilized. This results in saturation of only the central target area of the absorbent core and excessive thickness, bulkiness, and sagging of the wet, heavy absorbent material resulting in poor performance, product fit and wearer discomfort. US 6,437,214B1 disclosed an absorbent article which includes an absorbent core having multiple absorbent layers; the intake capability of the absorbent system is maintained by keeping a second layer of the absorbent system at low saturation. A problem with such an absorbent article is that incoming fluids are not properly absorbed and do not provide enough control of the incoming fluids to stop leakage from occurring.

US 2001/0014797 A1 discloses an absorbent incontinence pad provided with a liquid impervious air permeable back sheet and an absorbent unit partly covered by the back sheet, wherein the absorbent unit has a non-woven fabric substrate, an absorbent zone formed by a plurality of highly absorbent layers extending in the form of bands on the surface of the non-woven fabric substrate and an air permeable zone where no such highly absorbent layer exists.

US 2011/0270204 A1 describes a water-absorbent sheet composition containing a structure in which an absorbent layer containing a water-absorbent resin and an adhesive is sandwiched with two or more sheets of hydrophilic nonwoven fabrics.

EP 3025687 relates to an absorbent article in which a three-dimensional opening hole mesh sheet is combined with an air-laid absorber. In a liquid-permeable front surface sheet, a large number of small convex portions protruding to a skin side are formed. The absorber is an air-laid absorber that has a five-layer structure in which an air-laid pulp non-woven fabric layer, a high-absorbent polymer layer, an air-laid pulp non-woven fabric layer, a high-absorbent polymer layer and an air-laid pulp non-woven fabric layer are stacked sequentially from the side of the liquid-permeable front surface sheet.

WO99/17679 A2 describes an absorbent core having multiple absorbent layers, wherein the absorbent layers interact in such a manner which preferentially locates absorbed liquid in an appointed, high saturation wicking layer. The intake capability of the absorbent system is ensured by keeping a second layer of the absorbent system at low saturation levels. The low saturation in this layer provides void volume for the incoming insult as well as a high permeability, but the structure of the low saturation layer is also balanced to provide an appropriately high level of capillary tension. This low saturation layer is used in addition to a surge material and provides intake functionality in addition to that provided by the surge material.

The absorbent core of current absorbent articles does not adequately meet current performance objectives. Consequently, there remains a need for absorbent structures which can provide improved fluid uptake of liquids and improved liquid distribution to move liquids out of the target area to maintain this desirable liquid uptake behavior for the life of the product.

The present invention aims to resolve at least some of the problems mentioned above.

### SUMMARY OF THE INVENTION

The present invention concerns an absorbent article, preferably a disposable absorbent article, such as a diaper. In a first aspect, the present invention specifically concerns a multi-layer absorbent material according to claim 1. The differential concentration of superabsorbent polymer particles between the two absorbent layers presents the advantage that the incoming fluids can be efficiently absorbed in a two-fold way, firstly by the superabsorbent polymer particles (3) in said booster absorbent layer (26) and secondly by the superabsorbent polymer particles (28) in said central absorbent layer (18). Preferably the booster absorbent layer comprises a smaller surface area than the central absorbent layer, more preferably the booster absorbent layer comprises a surface area which is about the surface area of the ADL, preferably the booster absorbent layer and the ADL comprise surface areas which differ within about 50%, more preferably within 60%, still more preferably within 70%, even more preferably within 80%, yet more preferably within 90%, still even more preferably within 95% from one another.

A multi-layer absorbent material according to the invention provides an at least three-layered structure with very different properties. The role of the acquisition and distribution layer (6) is to better distribute the fluids, before accessing the underneath booster absorbent layer (26). The booster absorbent layer (26) is comprising superabsorbent polymer particles (3) that are preferably immobilized in a nonwoven carrier (2) comprising a top layer (17) consisting of staple fibers (31) penetrable by the superabsorbent polymer particles (3) and a nonwoven bottom layer (25) preferably hydroentangled to the top layer (17). Said booster absorbent layer (26) provides an increased absorption capacity within the "hot" zone of a diaper, i.e. the zone in a diaper where insults are most likely to occur. To some extent, the fluids are also distributed by that layer, via the capillary voids present between e.g. carrier fibers and SAP particles. Therefore, in a preferred embodiment, the booster layer comprises high-permeability SAPs. Such SAPs maintain the voids, even if the particles are swollen by the fluid.

In a second aspect, the present invention provides an absorbent article according to claim 8 comprising a multi-layer absorbent material according to any of claims 1 to 7.

In a third aspect, the invention also provides a process according to claim 9 for manufacturing a multi-layer absorbent material according to any of claims 1 to 7.

To further improve the desired balance of absorbent properties, there have been identified a number of important factors which can allow the absorbent layer regions to better work in combination, and thereby provide an improved overall system performance. The booster absorbent layer (26) works in tandem with the underneath central absorbent layer (18) because each of those layers comprises an amount of superabsorbent polymer particles (3 and 28) which is preferably between 1 g and 20 g, more preferably between 2 g and 15 g, still more preferably between 3 g and 10 g, e.g. 4 g, 5 g, 6 g, 7 g, 8 g, 9 g or any value there between such as about 6.5 g. According to the invention, the amount of SAP in said booster absorbent layer and the amount of SAP in said central absorbent layer differ by less than 50%, preferably by less than 40%, still more preferably by less than 30%, yet more preferably by less than 20%, even more preferably by less than 10%, most preferably said amounts of SAP are about the same.

The differential concentration of the superabsorbent polymer particles (3 and 28) within the two absorbent layers allows a better absorption of the incoming fluids and the superabsorbent polymer particles (3 and 28) are in a sufficient amount to absorb the incoming fluids.

Furthermore, the acquisition and distribution layer (6) and the booster absorbent layer (26) are placed towards the front edge of the central absorbent layer (18) to better distribute and absorb fluids in the frontal region of an absorbent article. The frontal region is the intake region of incoming fluids and allows an improved fluids intake function.

In a final aspect, the invention provides use according to claim 12 of a multi-layer absorbent material according to any of claims 1 to 7 for manufacturing an absorbent article.

The multi-layer absorbent material provided by the invention can be particularly advantageous for use in an absorbent article such as a diaper.

Preferred embodiments are as specified in the dependent claims.

### BRIEF DESCRIPTION OF THE DRAWINGS

The invention will be further described in detail with reference to the exemplary embodiments represented by the accompanying figures, in which
**FIG. 1** shows a general schematic cross-sectional view of the multi-layer structure of the absorbent material with a fluffless central absorbent layer (18) according to the present invention.
**FIG. 2** shows a general schematic cross-sectional view of a multi-layer structure of an absorbent material with a central absorbent layer (18) comprising fluff pulp and superabsorbent.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

As used herein, the following terms have the following meanings:
"Absorbent article" refers to devices that absorb and contain liquid, and more specifically, refers to devices that are placed against or in proximity to the body of the wearer to absorb and contain the various exudates discharged from the body.

Absorbent articles include but are not limited to diapers, adult incontinence briefs, training pants, diaper holders and liners, sanitary napkins and the like. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn.

The absorbent article of the present invention preferably comprises a liquid permeable topsheet, a liquid impermeable back sheet, and an absorbent medium disposed between the topsheet and the backsheet. The absorbent medium may comprise a booster absorbent layer and a central absorbent layer according to the present invention. The absorbent article may also include one or more features such as, but not limited to, ears or side panels, leg cuffs, fastener components and/or a belt. The top sheet, back sheet and the absorbent medium could be made from any suitable material known to the person skilled in the art.

"Acquisition and distribution layer" or "ADL", also refers to a sub-layer which preferably is a nonwoven wicking layer under the topsheet (or face fabric) of an absorbent product, which speeds up the transport and improves distribution of fluids throughout the absorbent core.

"Absorbent medium" is the absorbent structure disposed between the topsheet and the backsheet of the absorbent article in at least the crotch region of the absorbent article. The absorbent material can be of any conventional kind. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about 20 times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fibre, flake, sphere, etc. possesses a higher crosslink density than the inner portion of the superabsorbent. The superabsorbent materials may be in any form which is suitable for use in absorbent composites including particles, fibres, flakes, spheres, and the like.

Preferably, the absorbent medium comprises a nonwoven carrier according to the present invention.

"Diaper" refers to an absorbent article generally worn by infants and incontinent persons about the lower torso.

"Disposable" is used herein to describe articles that are generally not intended to be laundered or otherwise restored or reused (i. e. they are intended to be discarded after a single use and, preferably, to be recycled, composted or otherwise disposed of in an environmentally compatible manner).

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

"Nonwoven" refers to a manufactured sheet, web, or batt of directionally or randomly oriented fibres bonded by friction and/or cohesion and/or adhesion, excluding paper and products that are woven, knitted, tufted stitchbonded incorporating binding yarns or filaments, or felted by wet-milling, whether or not additionally needled. The fibres may be of natural or man-made origin. They may also be staple or continuous filaments or be formed in situ.

"Pulp fluff" or "fluff pulp" refers to a material made up of cellulose fibers. The fibers can be either natural or synthetic, or a combination thereof. The material is typically lightweight and has absorbent properties.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm. The staple fibers for use in the invention can be prepared from thin and/or coarse fibers, preferably polyester fibers. The thin fibers have a dtex below 3, preferably in the range of 1 to 3 whereas the coarse fibers have a dtex of at least 3 and preferably below 45 dtex. In case a blend of thin and coarse fibers is used, the coarse fibres preferably have a dtex value which is at least two times, but no greater than 15 times that of the thin fibers.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Topsheet" refers to a liquid permeable material sheet forming the inner cover of the absorbent article and which in use is placed in direct contact with the skin of the wearer. The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The "backsheet" refers to a material forming the outer cover of the absorbent article. The backsheet may be the same or different in different parts of the absorbent article. At least in the area of the absorbent medium, the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The term "density" or "concentration" when referring to the absorbent material, in particular the SAP, of a layer, refers to the amount of the absorbent material divided by the surface area of the layer over which the absorbent material is spread out.

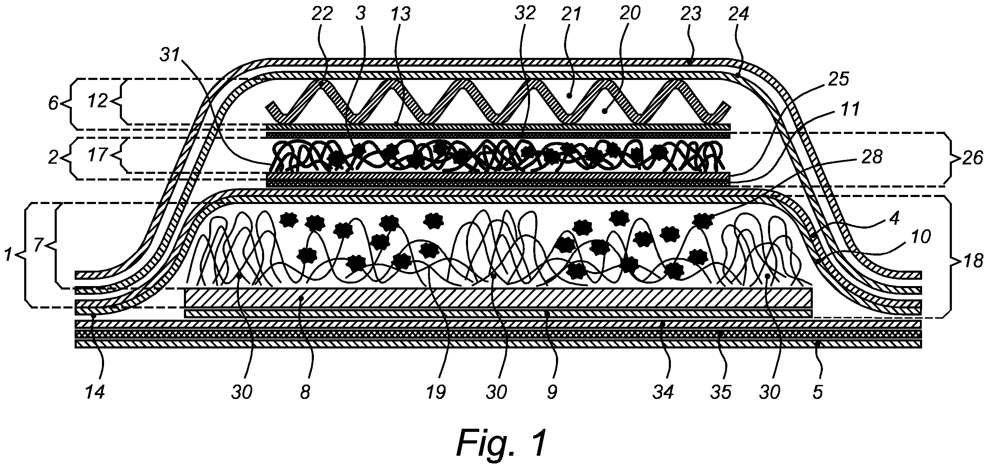

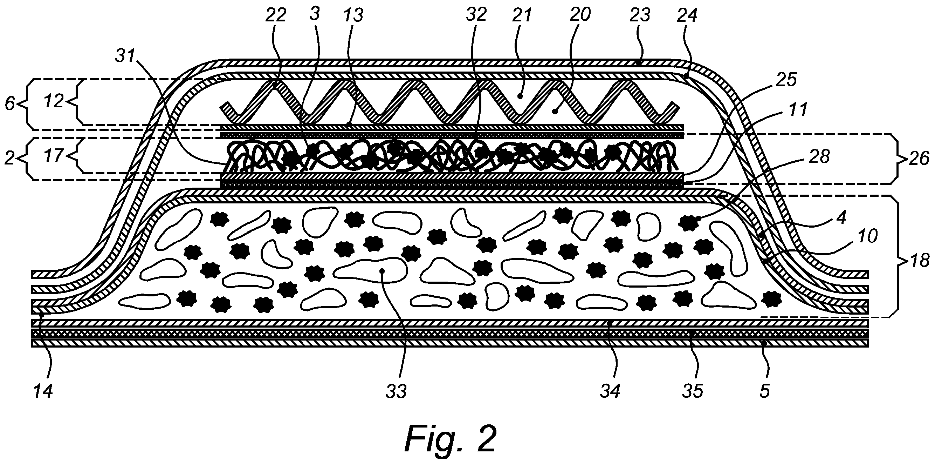

**FIG. 1** and **2** show two embodiments of an absorbent article, Fig. 1 being according to the present invention. **FIG. 1** is a general schematic cross-sectional view of the multi-layer structure of the absorbent material with a fluffless central absorbent layer (18) whereas **FIG. 2** is a general schematic cross-sectional view of the multi-layer structure of the absorbent material with a fluff pulp containing central absorbent layer (18).

The inventors have found a way to provide an improved absorbent core and process for making such a core.

In particular, the present invention provides in a first aspect a multi-layer absorbent material comprising: a topsheet (23), a backsheet (5), a central absorbent layer (18) positioned between the topsheet (23) and the backsheet (5), said central absorbent layer (18) comprising superabsorbent polymer particles (28), an acquisition and distribution layer (6) positioned between the topsheet (23) and the central absorbent layer (18) for receiving fluids from the topsheet (23) and transferring the fluids for absorption by the central absorbent layer (18), and a booster absorbent layer (26) positioned between the acquisition and distribution layer (6) and the central absorbent layer (18) (Fig.1). The booster absorbent layer (26) comprises a nonwoven carrier (2) with superabsorbent polymer particles (3) whereby the booster absorbent layer is comprising a higher concentration of superabsorbent polymer particles than the central absorbent layer. The differential concentration of superabsorbent polymer particles between the two absorbent layers presents the advantage that the incoming fluids can be more efficiently absorbed than in prior art multi-layer absorbent materials, due to a two-fold process, firstly by the superabsorbent polymer particles (3) in said booster absorbent layer (26) and secondly by the superabsorbent polymer particles (28) in said central absorbent layer (18) (Fig. 1). Furthermore, the booster layer can be placed at a region near a probable miction point. This allows to increase the absorbent capacity at this region without the need to provide an absorbent layer with location-dependent distribution of absorbent material, such as SAPs. Moreover, a higher concentration of SAP in the booster absorbent layer than in the central absorbent layer leads to a larger absorption capacity by the booster absorbent layer, which is preferably smaller than the central absorbent layer and positioned closer to the middle of the absorbent article, i.e. further away from the edges, hereby reducing the probability of leakage.

Therefore, preferably the booster absorbent layer is disposed mainly near a possible miction point, more preferably at least 50%, even more preferably at least 55%, still more preferably at least 60%, yet more preferably at least 65% of the booster absorbent layer is disposed in the front half of the absorbent article. The booster absorbent layer increases the absorbency at the region near possible miction points, which reduces the probability of leakage of fluids which could not be absorbed fast enough in prior art absorbent articles. Furthermore, fluids which pass through the booster absorbent layer without directly being absorbed, can be absorbed by the central absorbent layer. The above described situation occurs frequently as e.g. urine discharges usually result in a large amount of fluids being discharged in a small area and in a short period.

A further advantage of the present invention, is the ease of producing such an absorbent article whereby the absorbent capacity can be increased near a possible miction point, in particular by the presence of the at least two absorbent layers. The use of two layers, e.g. instead of one layer with differential absorption capacity, allows to produce absorbent articles of different types and of different sizes using the same components, methods and/or apparatuses.

In a preferred embodiment, the multi-layer absorbent material is comprising the central absorbent layer (18) as a first absorbent core layer of a first size, and at least one additional booster absorbent layer (26) overlying the first absorbent core layer and being of a different size than the first absorbent core layer thereby forming an absorbent multi-layer core. The booster absorbent layer is comprising a maximum length of between 5 cm and 50 cm, more preferably between 10 cm and 40 cm, yet more preferably between 20 cm and 30 cm and a maximum width of between 2.5 cm and 30 cm, more preferably between 5 cm and 20 cm, still more preferably between 7.5 cm and 15 cm. The central absorbent layer is comprising a maximum length of between 5 cm and 80 cm, more preferably between 15 cm and 65 cm, yet more preferably between 30 cm and 50 cm and a maximum width of between 2.5 cm and 50 cm, more preferably between 5 cm and 35 cm, still more preferably between 10 cm and 20 cm.

In a preferred embodiment, the booster absorbent layer (26) is comprising an amount of superabsorbent polymer particles (3) which is between 1 g and 30 g, more preferably between 2 g and 20 g, yet more preferably between 3 g and 10 g.

In a preferred embodiment, the central absorbent layer (18) is comprising an amount of superabsorbent polymer particles (28) which is between 1 g and 30 g, more preferably between 2 g and 20 g, yet more preferably between 3 g and 10 g.

According to the invention, the central and/or booster absorbent layer comprise superabsorbent particles which are distributed according to a pattern, preferably a pattern comprising areas (30) which are essentially free from SAPs, such as a clustered pattern.

According to the invention, the central absorbent layer (18) is comprising a fluffless absorbent layer. In a preferred embodiment, the central absorbent layer (18) is comprising a fluffless top layer (7) and superabsorbent polymer particles (28) (Fig.1). In Fig.2 not according to the invention, the central absorbent layer (18) is comprising a blend of fluff pulp (33) and superabsorbent polymer particles (28).

In the various aspects of the invention, the central absorbent layer (18) may comprise a fluffless top layer (7) or a fluff pulp (33). As in the previous aspects of the invention, these materials will be configured to provide maximum permeability while maintaining enough capillary tension to control the movement of the liquid and not allow leakage to occur. For example, the central absorbent layer (18) of the present invention incorporates nonwoven materials with superabsorbent polymer particles in the fluffless top layer (7).

The central absorbent layer (18) is preferably attached by thermoplastic adhesive (11) to the booster (26). Note that the central absorbent layer may be provided with a core wrap (4, 34) as indicated in figs. 1 and 2.

The central absorbent layer (18) is preferably wrapped between two layers of nonwoven (4, 34), the so-called core wrap. Or equivalently, the central absorbent layer preferably comprises a core wrap, which preferably comprise two layers of nonwoven (4, 34). The lower core wrap (34) can be attached to the bottom layer (8) of the nonwoven carrier (1) by thermoplastic adhesive (9). The upper core wrap (4) can be attached to the top surface of the nonwoven carrier (1) by thermoplastic adhesive (10). The superabsorbent polymer particles (28) can be partly in contact with the adhesive coating (10) on the upper core wrap (4).

In a preferred embodiment, the widths of the core wrap nonwovens exceed the width of the nonwoven carrier (1), so that the core wrap nonwovens can be bonded with each other along the side edges by thermoplastic adhesive (14 ). The core-wrapped central absorbent layer (18) can be attached to the back sheet (5), e.g. by a thermoplastic adhesive layer (35).

On top of the booster absorbent layer (26), an acquisition and distribution layer (6) is placed which is preferably attached by thermoplastic adhesive (32) to the booster absorbent layer (26). Preferably, the acquisition and distribution layer (6) is based upon Nonwovens Innovation & Research Institute's proprietary Hydrospace technology as described in EP 1644564A1 **(****FIGS. 1** and **2****).** Therefore, preferably, the ADL according to the present invention comprises a thin, 3D nonwoven spacer fabric with discrete, channel-like voids or cells within the fabric cross-section, which relies on fluid forces rather than conventional mechanical methods to periodically interconnect, e.g. hydro-entangle, fibres from at least two web structures which are separated by a spacer system during their production. Also preferably, the ADL comprises a nonwoven fabric, the fabric comprising at least two separate but interconnected layers, each of the layers being provided with discrete interconnections so as to provide discrete voids between the two layers of fabric. The shape of the voids may vary. However, preferentially, the voids comprise a channel and/or a tube, e. g. a plurality of channels and/or tubes within the structure of the fabric. The channels and/or tubes may be substantially cylindrical in shape. However, it will be understood by one skilled in the art that the size and/or shape of the voids may be influenced by the choice of the spacer material. Similarly, the size of the voids may vary, depending, inter alia, on the nature of the use of the nonwoven fabric. However, preferentially, the channels and/or tubes are such that they comprise a diameter in the range of from 0.2 mm to 8.5 mm, more preferably from 0.5 mm to 6 mm, yet more preferably from 1 mm to 5 mm, e.g. 2 mm, 3 mm, 4 mm or any value therebetween. The acquisition and distribution layer (6) can preferably be attached by thermoplastic adhesive (32) to the underneath booster absorbent layer (26). The multi-layer absorbent material being covered by a nonwoven topsheet (23) attached to the components underneath with a layer of thermoplastic adhesive (24). This is particularly preferred for absorbent articles comprising such an ADL or if such an ADL is to be used in an absorbent article.

Fluids are substantially guided in the longitudinal direction through the channels (21) and are further collected in the tubes (20) by e.g. capillary absorption that allows for the fluids to flow along the tubes (20). This promotes removal of a stationary portion of the fluids present on the nonwoven topsheet (23) and results in a better distribution of the fluids through the acquisition and distribution layer (6), before penetrating first the booster absorbent layer (26) and afterwards the underneath absorbent core (18).

In a preferred embodiment, the acquisition and distribution layer (6) is comprising a top layer (12) comprising staple fibers (22) and a, preferably essentially flat, non woven bottom layer (13), the acquisition and distribution layer (6) comprising an undulated structure. In a preferred embodiment, said top-layer (12) comprises corrugations which at least partially define said undulated channel structure. In a preferred embodiment, the ADL (6) comprises a maximum length of between 5 cm and 50 cm, more preferably between 10 cm and 40 cm, yet more preferably between 20 cm and 30 cm, and a maximum width of between 2.5 cm and 30 cm, more preferably between 5 cm and 20 cm, still more preferably between 7.5 cm and 15 cm. In a preferred embodiment, the undulated structure comprises two neighboring tubes (19) separated by a channel (21) and/or two neighboring channels (21) separated by a tube, whereby said two neighboring tubes and/or channels comprise a separating distance of between 3 mm and 20 mm, preferably between 5 mm and 15 mm, more preferably between 8 mm and 13 mm, still more preferably 9, 10, 11 or 12 mm, the distance being measured between the center of the neighboring tubes or channels (21), to improve fluids distribution, and a nonwoven bottom layer (13) **(****Figs. 1** and **2****).**

In a preferred embodiment, the acquisition and distribution layer (6) and the booster absorbent layer (26) are about the same size, preferably the length and/or width of the ADL differs from the length and/or width of the booster absorbent layer by less than 50%, more preferably by less than 40%, still more preferably by less than 30%, yet more preferably by less than 20%, even more preferably by less than 10%, still even more preferably by less than 5%.

In a second aspect, the invention provides an absorbent article according to claim 8 comprising a multi-layer absorbent material according to any of claims 1 to 7.

In a third aspect, the invention provides a process according to claim 9 for manufacturing a multi-layer absorbent material according to any of claims 1 to 7, comprising the steps of:
a) providing a topsheet (23) and a backsheet (5);
b) providing an acquisition and distribution layer (6);
c) providing an absorbent multi-layer core by:
   c1) providing a booster absorbent layer (26);
   c2) providing a central absorbent layer (18); and
d) disposing the absorbent multi-layer core between the top sheet (23) and the back sheet (5) and disposing the booster absorbent layer (26) between the acquisition and distribution layer (6) and the central absorbent layer (18),
thereby providing the multi-layer absorbent material.

In a preferred embodiment, the acquisition and distribution layer (6) and the booster absorbent layer (26) are placed mainly within the front half of the central absorbent layer (18) to distribute and absorb fluids, preferably at least 50%, more preferably at least 55%, still more preferably at least 60%, yet more preferably at least 65%, still even more preferably at least 70% of the ADL and/or the booster absorbent layer are disposed within the front half of the central absorbent layer.

In a preferred embodiment, the acquisition distribution layer (6), the nonwoven carrier (2), and the nonwoven carrier (1) are treated with a surfactant. The acquisition distribution layer (6), the nonwoven carrier (2), and the nonwoven carrier (1) can preferably be rendered hydrophilic through the use of a post treatment application of a surfactant. The hydrophilic characteristics of the acquisition distribution layer (6), the nonwoven carrier (2), and the nonwoven carrier (1) improve absorbent articles such as baby diapers and adult incontinence diapers by improving absorbency.

In a final aspect, the invention provides use according to claim 12 of a multi-layer absorbent material according according to any of claims 1 to 7 for manufacturing an absorbent article.

Although the present invention has been described with reference to preferred embodiments thereof, many modifications and alterations may be made by a person having ordinary skill in the art without departing from the scope of this invention which is defined by the appended claims.

## Claims

1. A multi-layer absorbent material comprising: a topsheet (23), a backsheet (5), a central absorbent layer (18) positioned between the topsheet (23) and the backsheet (5), said central absorbent layer (18) comprising superabsorbent polymer particles (28), an acquisition and distribution layer (6) positioned between the topsheet (23) and the central absorbent layer (18) for receiving fluids from the topsheet (23) and transferring the fluids for absorption to the central absorbent layer (18), and a booster absorbent layer (26) positioned between the acquisition and distribution layer (6) and the central absorbent layer (18), the booster absorbent layer (26) comprising a nonwoven carrier (2) with superabsorbent polymer particles (3), whereby the booster absorbent layer (26) comprises a higher concentration of superabsorbent polymer particles than the central absorbent layer (18), the concentration of superabsorbent polymer particles on a layer being defined as the amount of the absorbent material divided by the surface area of the layer over which the absorbent material is spread out,
**characterized in that** the central absorbent layer (18) is comprising a fluffless absorbent layer and superabsorbent polymer particles (28), **in that** the central and/or booster absorbent layer comprise superabsorbent polymer particles which are distributed according to a pattern, and **in that** the amount of superabsorbent polymer particles in the booster absorbent layer (26) and the amount of superabsorbent polymer particles in the central absorbent layer (18) differ by less than 50%.

2. The multi-layer absorbent material of claim 1 comprising the central absorbent layer (18) as a first absorbent core layer of a first size, and the booster absorbent layer (26) overlying the first absorbent core layer and being of a different size than the first absorbent core layer thereby forming an absorbent multi-layer core, whereby the booster absorbent layer is comprising a maximum length of between 5 cm and 50 cm, preferably between 10 cm and 40 cm, yet more preferably between 20 cm and 30 cm and a maximum width of between 2.5 cm and 30 cm, preferably between 5 cm and 20 cm, more preferably between 7.5 cm and 15 cm, and whereby the central absorbent layer is comprising a maximum length of between 5 cm and 80 cm, preferably between 15 cm and 65 cm, more preferably between 30 cm and 50 cm and a maximum width of between 2.5 cm and 50 cm, preferably between 5 cm and 35 cm, more preferably between 10 cm and 20 cm.

3. The multi-layer absorbent material according to any of the previous claims, wherein the booster absorbent layer (26) and/or the central absorbent layer (18) is comprising an amount of superabsorbent polymer particles which is between 1 g and 30 g, preferably between 2 g and 20 g, more preferably between 3 g and 10 g.

4. The multi-layer absorbent material according to any of the previous claims, wherein the amount of superabsorbent polymer particles in the booster absorbent layer (26) and the amount of superabsorbent polymer particles in the central absorbent layer (18) differ by less than 40%, preferably by less than 30%.

5. The multi-layer absorbent material according to any of the previous claims, wherein the central absorbent layer (18) is comprising a nonwoven carrier (1) with immobilized superabsorbent polymer particles (28).

6. The multi-layer absorbent material according to any of the previous claims, wherein the acquisition and distribution layer (6) is comprising a top layer (12) comprising staple fibers (22) and a, preferably flat, nonwoven bottom layer (13), the acquisition and distribution layer (6) comprising an undulated structure.

7. The multi-layer absorbent material according to any of the previous claims, wherein the acquisition and distribution layer (6) and the booster absorbent layer (26) each comprise a length and/or a width, whereby the length and/or width of the ADL differs from the length and/or width of the booster absorbent layer by less than 50%, preferably by less than 40%, more preferably by less than 30%, yet more preferably by less than 20%, even more preferably by less than 10%, still even more preferably by less than 5%.

8. An absorbent article comprising a multi-layer absorbent material according to any of claims 1 to 7.

9. Process for manufacturing a multi-layer absorbent material according to any of claims 1 to 7, comprising the steps of:
a) providing a topsheet (23) and a backsheet (5);
b) providing an acquisition and distribution layer (6);
c) providing an absorbent multi-layer core by:
c1) providing a booster absorbent layer (26);
c2) providing a central absorbent layer (18); and
d) disposing the absorbent multi-layer core between the topsheet (23) and the backsheet (5) and disposing the booster absorbent layer (26) between the acquisition and distribution layer (6) and the central absorbent layer (18),
thereby providing the multi-layer absorbent material.

10. Process according to claim 9, wherein at least 50%, preferably at least 55%, more preferably at least 60%, yet more preferably at least 65%, still even more preferably at least 70% of the ADL (6) and/or the booster absorbent layer (26) are disposed within the front half of the central absorbent layer (18).

11. Process according to any of the claims 9 to 10, wherein the booster absorbent layer (26) and/or the central absorbent layer (18) is comprising a nonwoven carrier (1) and a top layer (7) comprising staple fibers, the method further comprising the step of treating the top layer and the nonwoven carrier with a surfactant.

12. Use of a multi-layer absorbent material according to any of claims 1 to 7 for manufacturing an absorbent article.

13. Absorbent article according to claim 8, whereby the absorbent article is a diaper.

## Patentansprüche

1. Mehrschichtiges absorbierendes Material, umfassend: eine Decklage (23), eine Rückenlage (5), eine zentrale absorbierenden Schicht (18), die zwischen der Decklage (23) und der Rückenlage (5) angeordnet ist, wobei die zentrale absorbierende Schicht (18) superabsorbierende Polymerpartikel (28) umfasst, eine Aufnahme- und Verteilungsschicht (6), die zwischen der Decklage (23) und der zentralen absorbierenden Schicht (18) angeordnet ist, um Fluide von der Decklage (23) aufzunehmen und die Fluide zur Absorption an die zentrale absorbierende Schicht (18) zu übertragen, und eine verstärkende absorbierenden Schicht (26), die zwischen der Aufnahme- und Verteilungsschicht (6) und der zentralen absorbierenden Schicht (18) angeordnet ist, wobei die verstärkende absorbierende Schicht (26) einen Vliesträger (2) mit superabsorbierenden Polymerpartikeln (3) umfasst, wobei die verstärkende absorbierenden Schicht (26) eine höhere Konzentration an superabsorbierenden Polymerpartikeln umfasst als die zentrale absorbierenden Schicht (18), wobei die Konzentration an superabsorbierenden Polymerpartikeln auf einer Schicht als die Menge des absorbierenden Materials geteilt durch den Oberflächenbereich der Schicht, auf der das absorbierende Material ausgebreitet ist, definiert ist,
**dadurch gekennzeichnet, dass** die zentrale absorbierende Schicht (18) eine fusselfreie absorbierende Schicht und superabsorbierende Polymerpartikel (28) umfasst, dass die zentrale und/oder verstärkende absorbierende Schicht superabsorbierende Polymerpartikel umfassen, die gemäß einem Muster verteilt sind, und dass die Menge an superabsorbierenden Polymerpartikeln in der verstärkenden absorbierenden Schicht (26) und die Menge an superabsorbierenden Polymerpartikeln in der zentralen absorbierenden Schicht (18) um weniger als 50 % abweichen.

2. Mehrschichtiges absorbierendes Material nach Anspruch 1, das die zentrale absorbierende Schicht (18) als eine erste absorbierende Kernschicht einer ersten Größe, und die verstärkende absorbierende Schicht (26), die über der ersten absorbierenden Kernschicht liegt und eine andere Größe als die erste absorbierende Kernschicht aufweist, wodurch ein absorbierender mehrschichtiger Kern gebildet wird, wobei die verstärkende absorbierende Schicht eine maximale Länge zwischen 5 cm und 50 cm, vorzugsweise zwischen 10 cm und 40 cm, noch mehr bevorzugt zwischen 20 cm und 30 cm, und eine maximale Breite zwischen 2,5 cm und 30 cm, vorzugsweise zwischen 5 cm und 20 cm, noch mehr bevorzugt zwischen 7,5 cm und 15 cm umfasst, und wobei die zentrale absorbierende Schicht eine maximale Länge zwischen 5 cm und 80 cm, vorzugsweise zwischen 15 cm und 65 cm, noch mehr bevorzugt zwischen 30 cm und 50 cm und eine maximale Breite zwischen 2,5 cm und 50 cm, vorzugsweise zwischen 5 cm und 35 cm, noch mehr bevorzugt zwischen 10 cm und 20 cm aufweist.

3. Mehrschichtiges absorbierendes Material nach einem der vorhergehenden Ansprüche, wobei die verstärkende absorbierende Schicht (26) und/oder die zentrale absorbierende Schicht (18) eine Menge an superabsorbierenden Polymerpartikeln umfasst, die zwischen 1 g und 30 g, vorzugsweise zwischen 2 g und 20 g, mehr bevorzugt zwischen 3 g und 10 g liegt.

4. Mehrschichtiges absorbierendes Material nach einem der vorhergehenden Ansprüche, wobei die Menge an superabsorbierenden Polymerpartikeln in der verstärkenden absorbierenden Schicht (26) und die Menge an superabsorbierenden Polymerpartikeln in der zentralen absorbierenden Schicht (18) um weniger als 40 %, vorzugsweise um weniger als 30 % voneinander abweichen.

5. Mehrschichtiges absorbierendes Material nach einem der vorhergehenden Ansprüche, wobei die zentrale absorbierende Schicht (18) einen Vliesträger (1) mit immobilisierten superabsorbierenden Polymerpartikeln (28) umfasst.

6. Mehrschichtiges absorbierendes Material nach einem der vorhergehenden Ansprüche, wobei die Aufnahme- und Verteilungsschicht (6) eine obere Schicht (12) mit Stapelfasern (22) und eine vorzugsweise flache, nicht gewebte untere Schicht (13) umfasst, wobei die Aufnahme- und Verteilungsschicht (6) eine gewellte Struktur umfasst.

7. Mehrschichtiges absorbierendes Material nach einem der vorhergehenden Ansprüche, wobei die Aufnahme- und Verteilungsschicht (6) und die verstärkende absorbierende Schicht (26) jeweils eine Länge und/oder eine Breite aufweisen, wobei die Länge und/oder Breite der ADL von der Länge und/oder Breite der verstärkenden absorbierenden Schicht um weniger als 50 % abweicht, vorzugsweise um weniger als 40 %, mehr bevorzugt um weniger als 30 %, noch mehr bevorzugt um weniger als 20 %, noch mehr bevorzugt um weniger als 10 %, sogar noch mehr bevorzugt um weniger als 5 %.

8. Absorbierender Artikel, der ein mehrschichtiges absorbierendes Material nach einem der Ansprüche 1 bis 7 umfasst.

9. Verfahren zum Herstellen eines mehrschichtigen absorbierenden Materials nach einem der Ansprüche 1 bis 7, umfassend die Schritte:
a) Bereitstellen einer Decklage (23) und einer Rückenlage (5);
b) Bereitstellen einer Aufnahme- und Verteilungsschicht (6);
c) Bereitstellen eines absorbierenden mehrschichtigen Kerns durch:
c1) Bereitstellen einer verstärkenden absorbierenden Schicht (26);
c2) Bereitstellen einer zentralen absorbierenden Schicht (18); und
d) Anordnen des absorbierenden mehrschichtigen Kerns zwischen der Decklage (23) und der Rückenlage (5) und Anordnen der verstärkenden absorbierenden Schicht (26) zwischen der Aufnahme- und Verteilungsschicht (6) und der zentralen absorbierenden Schicht (18),
wodurch das mehrschichtige absorbierende Material bereitgestellt wird.

10. Verfahren nach Anspruch 9, wobei mindestens 50 %, vorzugsweise mindestens 55 %, mehr bevorzugt mindestens 60 %, noch mehr bevorzugt mindestens 65 %, sogar noch mehr bevorzugt mindestens 70 % des ADL (6) und/oder der verstärkenden absorbierenden Schicht (26) innerhalb der vorderen Hälfte der zentralen absorbierenden Schicht (18) angeordnet sind.

11. Verfahren nach einem der Ansprüche 9 bis 10, wobei die verstärkende absorbierende Schicht (26) und/oder die zentrale absorbierende Schicht (18) einen Vliesträger (1) und eine obere Schicht (7) aus Stapelfasern umfasst, wobei das Verfahren ferner den Schritt des Behandelns der oberen Schicht und des Vliesträgers mit einem Tensid umfasst.

12. Verwendung eines mehrschichtigen absorbierenden Materials nach einem der Ansprüche 1 bis 7 zur Herstellung eines absorbierenden Artikels.

13. Absorbierender Artikel nach Anspruch 8, wobei der absorbierende Artikel eine Windel ist.

## Revendications

1. Matériau absorbant multicouche comprenant : une feuille de dessus (23), une feuille de fond (5), une couche absorbante centrale (18) positionnée entre la feuille de dessus (23) et la feuille de fond (5), ladite couche absorbante centrale (18) comprenant des particules de polymère superabsorbant (28), une couche d'acquisition et de répartition (6) positionnée entre la feuille de dessus (23) et la couche absorbante centrale (18) permettant de recevoir des fluides provenant de la feuille de dessus (23) et de transférer les fluides pour absorption vers la couche absorbante centrale (18), et une couche absorbante d'appoint (26) positionnée entre la couche d'acquisition et de répartition (6) et la couche absorbante centrale (18), la couche absorbante d'appoint (26) comprenant un support non tissé (2) avec des particules de polymère superabsorbant (3), moyennant quoi la couche absorbante d'appoint (26) comprend une concentration plus élevée en particules de polymère superabsorbant que la couche absorbante centrale (18), la concentration en particules de polymère superabsorbant sur une couche étant définie comme la quantité du matériau absorbant, divisée par la superficie de la couche sur laquelle le matériau absorbant est étalé,
**caractérisé en ce que** la couche absorbante centrale (18) comprend une couche absorbante sans peluches et des particules de polymère superabsorbant (28), **en ce que** les couches absorbantes centrale et/ou d'appoint comprennent des particules de polymère superabsorbant qui sont réparties selon un motif, et **en ce que** la quantité de particules de polymère superabsorbant dans la couche absorbante d'appoint (26) et la quantité de particules de polymère superabsorbant dans la couche absorbante centrale (18) diffèrent de moins de 50 %.

2. Matériau absorbant multicouche selon la revendication 1 comprenant la couche absorbante centrale (18) en guise de première couche d'âme absorbante d'une première taille, et la couche absorbante d'appoint (26) recouvrant la première couche d'âme absorbante et étant d'une taille différente de la première couche d'âme absorbante en formant de ce fait une âme absorbante multicouche, moyennant quoi la couche absorbante d'appoint comprend une longueur maximale comprise entre 5 cm et 50 cm, de préférence entre 10 cm et 40 cm, encore plus préférablement entre 20 cm et 30 cm et une largeur maximale comprise entre 2,5 cm et 30 cm, de préférence entre 5 cm et 20 cm, plus préférablement entre 7,5 cm et 15 cm, et moyennant quoi la couche absorbante centrale comprend une longueur maximale comprise entre 5 cm et 80 cm, de préférence entre 15 cm et 65 cm, plus préférablement entre 30 cm et 50 cm et une largeur maximale comprise entre 2,5 cm et 50 cm, de préférence entre 5 cm et 35 cm, plus préférablement entre 10 cm et 20 cm.

3. Matériau absorbant multicouche selon l'une quelconque des revendications précédentes, dans lequel la couche absorbante d'appoint (26) et/ou la couche absorbante centrale (18) comprennent une quantité de particules de polymère superabsorbant qui est entre 1 g et 30 g, de préférence entre 2 g et 20 g, plus préférablement entre 3 g et 10 g.

4. Matériau absorbant multicouche selon l'une quelconque des revendications précédentes, dans lequel la quantité de particules de polymère superabsorbant dans la couche absorbante d'appoint (26) et la quantité de particules de polymère superabsorbant dans la couche absorbante centrale (18) diffèrent de moins de 40 %, de préférence de moins de 30 %.

5. Matériau absorbant multicouche selon l'une quelconque des revendications précédentes, dans lequel la couche absorbante centrale (18) comprend un support non tissé (1) avec des particules de polymère superabsorbant (28) immobilisées.

6. Matériau absorbant multicouche selon l'une quelconque des revendications précédentes, dans lequel la couche d'acquisition et de répartition (6) comprend une couche supérieure (12) comprenant des fibres discontinues (22) et une couche inférieure non tissée (13), de préférence plate, la couche d'acquisition et de répartition (6) comprenant une structure ondulée.

7. Matériau absorbant multicouche selon l'une quelconque des revendications précédentes, dans lequel la couche d'acquisition et de répartition (6) et la couche absorbante d'appoint (26) comprennent chacune une longueur et/ou une largeur, moyennant quoi la longueur et/ou la largeur de la couche de recueil et répartition diffèrent par rapport à la longueur et/ou à la largeur de la couche absorbante d'appoint de moins de 50 %, de préférence de moins de 40 %, plus préférablement de moins de 30 %, encore plus préférablement de moins de 20 %, même plus préférablement de moins de 10 %, encore même plus préférablement de moins de 5 %.

8. Article absorbant comprenant un matériau absorbant multicouche selon l'une quelconque des revendications 1 à 7.

9. Processus permettant de fabriquer un matériau absorbant multicouche selon l'une quelconque des revendications 1 à 7, comprenant les étapes consistant à :
a) fournir une feuille de dessus (23) et une feuille de fond (5) ;
b) fournir une couche d'acquisition et de répartition (6) ;
c) fournir une âme absorbante multicouche par :
c1) la fourniture d'une couche absorbante d'appoint (26) ;
c2) la fourniture d'une couche absorbante centrale (18) ; et
d) disposer l'âme absorbante multicouche entre la feuille de dessus (23) et la feuille de fond (5) et disposer la couche absorbante d'appoint (26) entre la couche d'acquisition et de répartition (6) et la couche absorbante centrale (18),
en fournissant de ce fait le matériau absorbant multicouche.

10. Processus selon la revendication 9, dans lequel au moins 50 %, de préférence au moins 55 %, plus préférablement au moins 60 %, encore plus préférablement au moins 65 %, encore même plus préférablement au moins 70 % de la couche de recueil et répartition (6) et/ou de la couche absorbante d'appoint (26) sont disposés au sein de la moitié avant de la couche absorbante centrale (18).

11. Processus selon l'une quelconque des revendications 9 à 10, dans lequel la couche absorbante d'appoint (26) et/ou la couche absorbante centrale (18) comprennent un support non tissé (1) et une couche supérieure (7) comprenant des fibres discontinues, le procédé comprenant en outre l'étape de traitement de la couche supérieure et du support non tissé avec un agent tensioactif.

12. Utilisation d'un matériau absorbant multicouche selon l'une quelconque des revendications 1 à 7 pour la fabrication d'un article absorbant.

13. Article absorbant selon la revendication 8, moyennant quoi l'article absorbant est une couche.
